# EUROPEAN PATENT APPLICATION

(11) **EP 4 527 849 A1**
(43) Date of publication of application: **26.03.2025**
(21) Application number: 23198276.0
(22) Date of filing: 19.09.2023
(51) Int. Cl.: C07K 14/725, A61K 39/00, A61P 35/00

(54) **HLA-INDEPENDENT ANTIGEN BINDERS, SUCH AS T-CELL RECEPTORS, AGAINST PD-L1**

(71) Applicant: Universitätsmedizin der Johannes Gutenberg-Universität Mainz, 55131 Mainz (DE); Universität Duisburg-Essen, 45141 Essen (DE)
(72) Inventor: Fatho, Martina, 55268 Wörrstadt (DE); Wölfel, Catherine, 55128 Mainz (DE); Wölfel, Thomas, 55128 Mainz (DE); Paschen, Annette, 45122 Essen (DE); Schadendorf, Dirk, 45122 Essen (DE)
(74) Representative: Krauss, Jan

(57) **Abstract**

The present invention relates to novel tumor-associated antigens, which elicit a T-cell response independently from a presentation by MHC. PD-L1 was found to be a target of CD8-positive T-cell clones which could detect their target antigen on the surface of HLA I-negative melanoma cells. Thus, the invention provides proteins, protein fragments and polypeptides of the novel antigens for use in medicine, for example for the treatment, diagnosis and prevention of a tumor disease. Also provided are nucleic acids expressing the antigens of the invention, binding agents specific for the antigens of the invention, such as T-cell receptor chains and isolated T cells which are reactive against the antigens of the invention or which express the T-cell receptors of the invention. The invention further pertains to pharmaceutical compositions, especially vaccine compositions, comprising the antigens, nucleic acids, binding agents or T cells in accordance with the invention, and methods for the generation of T cells specifically reactive to the antigens of the invention in an MHC-independent manner.

## Description

The present invention relates to novel tumor-associated antigens, which elicit a T-cell response independently from a presentation by MHC. PD-L1 was found to be a target of CD8-positive reactive T-cell clones that could detect their target antigen on the surface of HLA I-negative melanoma cells. Thus, the invention provides proteins, protein fragments and polypeptides of the novel antigens for use in medicine, for example for the treatment, diagnosis and prevention of a tumor disease. Also provided are nucleic acids expressing the antigens of the invention, binding agents specific for the antigens of the invention, such as T-cell receptor chains and isolated T cells which are reactive against the antigens of the invention or which express the T-cell receptors of the invention. The invention further pertains to pharmaceutical compositions, especially vaccine compositions, comprising the antigens, nucleic acids, binding agents or T cells in accordance with the invention, and methods for the generation of T cells specifically reactive to the antigens of the invention in an MHC-independent manner.

### Background of the invention

One of the major mechanisms by which cancer cells evade the immune system is via down regulation and loss of their major histocompatibility complex class I (MHC-I) or II (MHC-II) molecules (e.g. Stupia et al., Clin Cancer Res 2023, PMID: 37199727, DOI: 10.1158/1078-0432.CCR-23-0099) (aka human leukocyte antigens (HLAs)). Normally, MHC-1-positive tumor cells would be targeted by T-cells with T-cell receptors (TCRs) recognizing tumor-specific peptides displayed by the MHC class-I molecules. The recognition and binding of tumor-cell surface peptide-loaded MHCs (pMHCs) by TCRs results in the formation of a cytolytic synapse between the T-cell and cancer cell, leading to the directed massive release of cytotoxic proteins such as perforin and granzymes, as well as clonal T-cell activation and proliferation. Optimal activation of the T cells in this and other synaptic interactions requires two signals, the TCR-MHC interaction, known as "Signal 1" and a costimulatory signal ("Signal 2") through one of several costimulatory receptors on T cells (e.g., CD28, CD137, OX40, CD27, ICOS, GITR) and their cognate ligands (e.g., CD80/86, CD137L, OX40L, CD70, ICOS-L, GITR-L) on the targeted cells or professional antigen-presenting cell (APC). A third signal, production of immunostimulatory cytokines, helps to drive T-cell differentiation and expansion. The MHC-1 loss-based mechanism of tumor escape is further complicated by the fact that tumor-cell specific neoantigens are often "minimal" or difficult to discriminate because they may only be single-residue different from their wild-type counterpart, low affinity or not presented well by MHC-I.

Loss or downregulation of the MHC-I molecules and absence of strong tumor antigens in cancer cells allow those cells to escape recognition and killing by tumor-infiltrating T cells which are key components of anti-tumor immunological responses. Additionally, loss of costimulatory molecules (e.g., CD86, CD54), overproduction of checkpoint inhibitory molecules (e.g., PD-1, CTLA4) and tumor production of the tryptophan degrading-enzyme indoleamine 2,3-dioxygenase (IDO), which eliminates tryptophan, a key amino acid required for T-cell proliferation, are other examples of mechanisms utilized by tumors to evade cytotoxic T cells.

Today, various therapeutic strategies seek to harness the killing power of T cells in a TCR functionality-independent manner, bypassing the limitation of HLA-restricted antigen recognition. Two of the most important TCR function-independent T cell-based therapeutic strategies employed today are T-cell redirecting bispecific antibodies (TRBAs) and chimeric antigen receptor (CAR)-T cells.

Chimeric antigen receptors (CARs) engage antigen independently of HLA and enable sustained T-cell proliferation when they are endowed with both activating and costimulatory functions. Nevertheless, while remission rates have been noticeably elevated in numerous clinical trials targeting CD 19, CD22 or BCMA, relapses are still common.

With TRBAs the epsilon (ε) domain of cluster of differentiation 3 (CD3), a component of the TCR complex, is targeted with one combining (i.e., binding) domain, while a second binding domain (hence, "bispecific" antibody) binds a tumor-cell surface antigen. These TRBAs function to bring the T cells and targeted cells into close proximity to form a cytolytic synapse resulting in tumor-cell death. In the case of CAR-T cells, a cancer-cell surface antigen-targeting antibody fragment, fused to T-cell activating intracellular domains, is expressed as a neoreceptor on the surface of the T cells. These tumor antigen-recognizing "armed" T-cells will then identify, bind and kill the targeted cancer cells. Both of these strategies rely on antibodies to replace the function of the TCR, making them independent of the TCR and its cognate MHC-I/peptide recognition and both can be employed to recognize and target tumor-specific antigens outside the realm of MHC-1-displayed neoantigen peptides (Strohl WR, Naso M. Bispecific T-Cell Redirection versus Chimeric Antigen Receptor (CAR)-T Cells as Approaches to Kill Cancer Cells. Antibodies (Basel). 2019 Jul 3;8(3):41. doi: 10.3390/antib8030041. PMID: 31544847; PMCID: PMC6784091).

Hazini et al. (in: Deregulation of HLA-I in cancer and its central importance for immunotherapy. J Immunother Cancer. 2021 Aug;9(8):e002899. doi: 10.1136/jitc-2021-002899. PMID: 34353849; PMCID: PMC8344275) consider the mechanisms by which HLA function may be lost in clinical disease. They assess the implications for current immunotherapy approaches using checkpoint inhibitors and examine the prognostic impact of HLA loss demonstrated in clinical trials so far. Finally, they propose strategies that might be explored for possible patient stratification.

Programmed death-ligand 1 (PD-L1) also known as cluster of differentiation 274 (CD274) or B7 homolog 1 (B7-H1) is a protein that in humans is encoded by the *CD274* gene. PD-L1 (SEQ ID NO: 7) binds to its receptor PD-1, found on activated T cells, B cells, and myeloid cells, to modulate activation or inhibition. The PD-1-binding domain of the human PD-L1 is the Ig V-type PD-L1 domain between amino acids 19 and 127.

Immunotherapeutics, like most cancer drugs, can cause serious undesired side effects. Checkpoint inhibitor antibodies have become the most frequently applied type of immunotherapy in the clinic and also the most widely investigated immunotherapy approach in preclinical studies. Inhibiting antibodies against immune checkpoint molecules, such as PD-1/PDL-1 or CTLA-4, elicit impressive responses in a series of tumor entities. There is growing evidence that tumors with a high mutation load are particularly responsive to checkpoint blockade. Indeed, checkpoint inhibitors can boost T-cell reactivity against neoantigens generated by somatic mutations. However, a preexisting anti-tumoral T-cell immunity is required for these immunomodulatory antibodies to show an effect.

WO2014091034A1 relates to tumor-associated antigens, that elicit independently from a presentation via MHC a CD8-positive T-cell response. GM-CSF-Receptor alpha chain (CSF2RA) and Tyrosinase-related protein 2 (TRP-2) were found to be targets of CD8-positive T-cell clones which could detect the proteins on the surface of HLA I-negative melanoma cells. Thus, the application provides proteins, protein fragments and polypeptides of the novel antigens for use in medicine, for example for the treatment, diagnosis and prevention of a tumor disease.

US20190048083A1 relates to novel regulatory T-cell proteins. One protein, designated PD-L3, resembles members of the PD-L1 family, and co-stimulates αCD3 proliferation of T cells in vitro.

WO2013056716A1 discloses vaccine compositions comprising PD-L1 or peptide fragments thereof that are capable of eliciting immune responses useful in treatment of cancer, autoimmune diseases or infectious diseases.

Enhancing or generating T-cell responses against HLA-independently recognizable antigens would circumvent immune escape due to HLA loss and thus represent a substantial expansion of the immunotherapeutic repertoire. Thus, there is a need to provide targets leading to HLA-independent anti-tumor alpha/betaT-cell responses in patients with HLA-negative disease.

It is therefore an object of the present invention to provide novel HLA-independent antigens recognized by cytotoxic T-lymphocytes (CTL) that are stably expressed in a high proportion of tumors and is therefore suitable for a broad application. It is a further object of this invention to provide HLA-independent T-cell receptors (TCRs) against these antigens, as well as compositions comprising the TCRs, as well as uses thereof. Other objects of the present invention will become apparent to the person of skill when studying the specification of the present invention.

In a first aspect thereof, the object of the present invention is solved by providing a pharmaceutical composition comprising PD-L1 of SEQ ID NO: 7 or a functional homologue thereof at least 75% identical thereto or an immunogenically active peptide fragment comprising a consecutive sequence of at least 8 amino acids of PD-L1 or said functional homologue thereof, preferably of the PD-1-binding domain, or a nucleic acid encoding said PD- L1, said functional homologue thereof or said peptide fragment; and b) at least one pharmaceutically acceptable carrier for use in the prevention or treatment of a cancer cell exhibiting down-regulation or loss of HLA expression. Preferred is the pharmaceutical composition for use according to the present invention, which is an anti-cancer vaccine, and/or wherein said prevention or treatment comprises inducing a major histocompatibility complex (MHC)-independent T-cell response, preferably an MHC class I-independent T cell response, or an MHC class I- and II-independent T-cell response.

In a second aspect thereof, the object of the present invention is solved by providing a binding agent comprising the amino acid sequence of binding regions of a T-cell receptor binding to cancer cells expressing PD-L1 (SEQ ID No. 7) or a protein having at least 75% sequence identity to PD-L1, wherein said binding is independently from HLA. Preferred is the binding agent according to the present invention, wherein said T-cell receptor is selected from a nucleic acid sequence encoding a T-cell receptor expressed by one of the CTL clones 11C/25, 11C/34 and 5C/169 according to SEQ ID NO: 2, 4, or 6, or an amino acid sequence of a TCR alpha or beta chain selected from a sequence according to SEQ ID NO: 1, 3, or 5. In a preferred embodiment, the binding agent according to the present invention is selected from a TCR, a modified TCR, such as a chimeric T cell receptor comprising a murinized constant region and a human variable region, a single-chain TCR molecule, and a bispecific molecule, such as a bispecific antibody or a CAR.

The present invention further provides a nucleic acid molecule encoding for a binding agent according to the present invention, or a vector comprising the nucleic acid molecule, such as, for example, an expression vector comprising and expressing the nucleic acid molecule. The present invention further provides a recombinant or genetically modified cell comprising a nucleic acid molecule or vector according to the present invention. Preferably, the recombinant or genetically modified cell according to the present invention is a T cell expressing a TCR that binds to PD-L1 preferably to the PD-1-binding domain or a protein having at least 75% sequence identity to PD-L1 or of the PD-1-binding domain, wherein said binding is independently from HLA, such as, for example, a TCR expressed by one of the CTL clones 11C/25, 11C/34 or 5C/169 or a PD-L1-binding fragment thereof.

In a third aspect thereof, the object of the present invention is solved by providing an *in-vitro* method for generating HLA independent T cells, comprising a) providing a first cell that expresses PD-L1 (SEQ ID No. 7) or a protein having at least 75% sequence identity to PD-L1 or an immunogenic protein fragment thereof, preferably wherein the polypeptide is full length PD-L1 or the PD-1-binding domain, b) bringing a population of peripheral blood mononuclear cells (PBMCs) into contact with said first cell, and thereby stimulating said PBMCs, and c) selecting from the population of stimulated PBMCs T cells that have the ability to recognize a cell expressing the PD-L1 or an immunogenic protein fragment thereof used in a) independently of the expression of HLA in said cell.

In a fourth aspect thereof, the object of the present invention is solved by providing an HLA-independent T cell, generated according to a method according to the present invention.

In a fifth aspect thereof, the object of the present invention is solved by providing a pharmaceutical composition, comprising a binding agent according to the present invention, a nucleic acid or vector according to the present invention, a cell according to the present invention, or a T-cell according to the present invention, and at least one pharmaceutically acceptable carrier. Preferred is the pharmaceutical composition according to the present invention for use as a medicament, preferably for use in the prevention or treatment of a cancer cell exhibiting down-regulation or loss of HLA expression. Further preferred is the pharmaceutical composition for use according to the present invention, wherein said prevention or treatment comprises inducing a major histocompatibility complex (MHC)-independent T-cell response, preferably an MHC class I-independent T cell response, or an MHC class I- and II-independent T-cell response. Also preferred is the pharmaceutical composition for use according to the present invention, wherein the cancer cell expresses PD-L1 (SEQ ID No. 7) or a protein having at least 75% sequence identity to PD-L1, or of the PD-1-binding domain thereof.

In a sixth aspect thereof, the object of the present invention is solved by providing a method for eliciting a CD4- or CD8-positive T-cell response in a subject independently from a presentation of an antigen by MHC, comprising administering to said subject the pharmaceutical composition for use according to the present invention or the pharmaceutical composition according to the present invention.

In a seventh aspect thereof, the object of the present invention is solved by providing a kit comprising: a) the pharmaceutical composition for use according to the present invention or the pharmaceutical composition according to the present invention, and b) a composition comprising at least one second active ingredient.

In an eighth aspect thereof, the object of the present invention is solved by providing a method of preventing or treating a cancer cell exhibiting down-regulation or loss of HLA expression, wherein the cancer cell furthermore expresses PD-L1 (SEQ ID No. 7) or a protein having at least 75% sequence identity to PD-L1 or of the PD-1-binding domain thereof, the method comprising administering to subject suffering or prospectively suffering from said cancer an effective amount of the pharmaceutical composition for use according to the present invention, or of the pharmaceutical composition according to the present invention. Preferred is the method according to the present invention, wherein said prevention or treatment comprises inducing a major histocompatibility complex (MHC)-independent T-cell response, preferably an MHC class I-independent T-cell response, or an MHC class I- and II-independent T-cell response. Further preferred is the method according to the present invention, wherein the cancer cell is from a tumor carrying or expressing PD-L1, e.g. melanoma or a glioblastoma.

The present inventors have observed the emergence of HLA-independent anti-tumor alpha/betaT-cell responses in patients with HLA-negative disease. All known anti-PD-L1 effector molecules are derived from monoclonal antibodies with an inherent limitation of antigen sensitivity compared to T cell/TCR recognition. However, there is currently no definite answer to the question of whether TCRs have a clinically visible therapeutic advantage over CAR or comparable constructs. The different nature of their ligands (HLA/peptide complexes versus intact surface molecules) makes a direct comparison very difficult. In a first approach to this difficult question, Mansilla-Soto et al. compared CAR and so-called HIT receptors, i.e. artificial HLA-independent TCRs. They reported that HIT receptors offer advantages over CAR in therapeutic targeting of surface antigens with low expression levels (Mansilla-Soto et al., 2022).

It is therefore of importance to provide an HLA-independent antigen recognized by CTL that is stably expressed in a high proportion of tumors and is therefore suitable as target for a broad anti-cancer approach.

In the context of the present invention, the inventors surprisingly found that the protein PD-L1 is an antigen expressed by cancer cells that is recognized by CTLs independently of HLA presentation and expression. HLA-independent PD-L1-reactive T cells therefore lyse completely HLA-negative tumor-cell variants.

PD-L1 is already a well-established therapeutic target in oncology. Currently, several monoclonal antibodies against PD-L1 are approved for clinical use as immune checkpoint inhibitors in a variety of diseases. In contrast to PD-1 inhibitors, all are IgG1 antibodies characterized by high FcyR binding affinity, which in turn is associated with a highly enhanced capacity for ADCC (antibody-dependent cell-mediated cytotoxicity) and ADCP (antibody-dependent cellular phagocytosis) and a moderately enhanced capacity for CDC (complement-dependent cytotoxicity). Preservation of Fc effector function in IgG1 isotypes is thought to enhance the therapeutic efficacy of anti-PD-L1 antibodies through direct anti-tumor effects in addition to their indirect effects as immune checkpoint inhibitors.

Because successful immune checkpoint blockade ultimately releases preformed intact T-cell responses, it is effective only in a subset of patients. Several groups have therefore developed anti-PD-L1-CAR constructs, BiTE molecules, or nanobodies that directly kill tumor cells but also target the tumor microenvironment, particularly tumor-associated macrophages (TAMs). These constructs are in preclinical or early clinical testing (Li et al., 2022; Liu et al., 2020; Jiang et al., 2021; Horn et al., 2017; Khalique et al., 2021). All these anti-PD-L1 effector molecules originate from monoclonal antibodies with an inherent limitation of antigen sensitivity as compared to TCR recognition.

Promising data have been reported on an immunomodulatory vaccination of patients with a PD-L1 peptide, together with an IDO peptide and checkpoint blockade with an anti PD-1 antibody). Based on the data from this phase I/II study, the FDA has granted a breakthrough therapy designation for this treatment combination. But non-responsiveness in a particular patient, whose tumor was found to have lost HLA I, led to the assumption that this strategy would fail in HLA-negative disease (Kjeldsen et al., 2021).

The present invention relates to the following items:
Item 1: A pharmaceutical composition comprising PD-L1 of SEQ ID NO: 7 or a functional homologue thereof at least 75% identical thereto or an immunogenically active peptide fragment comprising a consecutive sequence of at least 8 amino acids of PD-L1 or said functional homologue thereof, preferably of the PD-1-binding domain thereof, or a nucleic acid encoding said PD-L1, said functional homologue thereof or said peptide fragment; and b) at least one pharmaceutically acceptable carrier for use in the prevention or treatment of a cancer cell exhibiting down-regulation or loss of HLA expression.
Item 2. The pharmaceutical composition for use according to Item 1 which is an anti-cancer vaccine.
Item 3. The pharmaceutical composition for use according to Item 1 or 2, wherein said prevention or treatment comprises inducing a major histocompatibility complex (MHC)-independent T-cell response, preferably an MHC class I-independent T-cell response, or an MHC class I- and II-independent T-cell response.
Item 4. The pharmaceutical composition for use according to any one of Items 1 to 3, wherein the cancer cell expresses PD-L1 (SEQ ID No. 7) or a protein having at least 75% sequence identity to PD-L1.
Item 5. The pharmaceutical composition for use according to any one of Items 1 to 4, wherein the cancer cell is from a tumor carrying or expressing PD-L1, e.g. melanoma or glioblastoma.
Item 6. The pharmaceutical composition for use according to any one of Items 2 to 5, wherein the vaccine composition further comprises an immunogenic protein which is not PD-L1 or an immunogenically active peptide fragment thereof.
Item 7. The pharmaceutical composition for use according to any one of Items 2 to 6, wherein the adjuvant is selected from the group consisting of bacterial DNA-based adjuvants, oil/surfactant based adjuvants, viral dsRNA-based adjuvants and imidazochinilines, or a Montanide ISA adjuvant.
Item 8. The pharmaceutical composition for use according to any one of Items 2 to 7, wherein the vaccine composition comprises antigen-presenting cells comprising the immunogenically active peptide fragment or a nucleic acid encoding said immunogenically active peptide fragment, wherein preferably the antigen-presenting cell is a dendritic cell.
Item 9. A binding agent comprising the amino acid sequence of a binding region of a T-cell receptor binding to a cancer cell expressing PD-L1 (SEQ ID No. 7) or a protein having at least 75% sequence identity to PD-L1, preferably of the PD-1-binding domain thereof, wherein said binding is independent of HLA.
Item 10. The binding agent according to Item 9, wherein said T-cell receptor is selected from a T-cell receptor expressed by one of the CTL clones 11C/25, 11C/34 and 5C/169, or an amino acid sequence of a TCR alpha or beta chain selected from a sequence according to SEQ ID NO: 1, 3, and 5.
Item 11. The binding agent according to Item 9 or 10, wherein said binding agent is selected from a TCR, a modified TCR, such as a chimeric T-cell receptor comprising murine constant regions and human variable regions, a single-chain TCR molecule, and a bispecific molecule, such as a bispecific antibody or a CAR.
Item 12. A nucleic acid molecule encoding a binding agent according to any one of Items 9 to 11, a vector comprising the nucleic acid molecule, such as, for example, an expression vector comprising and expressing the nucleic acid molecule.
Item 13. A recombinant or genetically modified cell comprising a nucleic acid molecule or vector according to Item 12.
Item 14. The recombinant or genetically modified cell according to Item 13, which is a T cell expressing a TCR that binds to PD-L1 or a protein having at least 75% sequence identity to PD-L1, preferably of the PD-1-binding domain thereof, wherein said binding is independently from HLA, such as, for example, a TCR expressed by one of the CTL clones 11C/25, 11C/34 or 5C/169 or a PD-L1 binding fragment thereof.
Item 15. An *in-vitro* method for generating HLA-independent T cells, comprising
   a) providing a first cell that expresses PD-L1 (SEQ ID No. 7) or a protein having at least 75% sequence identity to PD-L1 or an immunogenic protein fragment thereof, preferably wherein the polypeptide is full length PD-L1 or preferably the PD-1-binding domain,
   b) bringing a population of peripheral blood mononuclear cells (PBMCs) into contact with said first cell, and thereby stimulating said PBMCs, and
   c) selecting from the population of stimulated PBMCs T cells that have the ability to recognize a cell expressing the PD-L1 or an immunogenic protein fragment thereof used in a) independently of the expression of HLA in said cell.
Item 16. The method according to Item 15, wherein in step c) the ability of a T cell to recognize a cell expressing the PD-L1 or an immunogenic protein fragment thereof used in a) independently of the expression of HLA in said cell is determined by testing the reactivity of said T-cell against a cell expressing the PD-L1 or an immunogenic protein fragment thereof, wherein
   i) the cell expressing the PD-L1 or an immunogenic protein fragment thereof lacks MHC class I or MHC class I and II expression, and/or
   ii) the T-cell is tested for its reactivity against said cell expressing the PD-L1 or an immunogenic protein fragment thereof in the presence of antibodies against MHC class I or II; and/or
   iii) the T-cell is tested for its reactivity against xenogenic cells transfected with DNA or RNA encoding the PD-L1 or an immunogenic protein fragment thereof,
   wherein in i), ii) and/or iii) a T-cell that shows reactivity is a T-cell having the ability to recognize a cell expressing the PD-L1 or an immunogenic protein fragment thereof used in a) independent of the expression of HLA in said cell.
Item 17. An HLA independent T cell, generated according to a method according to Item 15 or 16.
Item 18. A pharmaceutical composition, comprising a binding agent according to any one of Items 9 to 11, a nucleic acid or vector according to Item 9, a cell according to Item 13 or 14, or a T-cell according to Item 17, and at least one pharmaceutically acceptable carrier.
Item 19. The pharmaceutical composition according to Item 18 for use as a medicament, preferably for use in the prevention or treatment of a cancer cell exhibiting down-regulation or loss of HLA expression.
Item 20. The pharmaceutical composition for use according to Item 18 or 19, wherein said prevention or treatment comprises inducing a major histocompatibility complex (MHC)-independent T-cell response, preferably an MHC class I-independent T-cell response, or an MHC class I- and II-independent T-cell response.
Item 21. The pharmaceutical composition for use according to any one of Items 18 to 20, wherein the cancer cell expresses PD-L1 (SEQ ID No. 7) or a protein having at least 75% sequence identity to PD-L1, preferably the PD-1-binding domain thereof.
Item 22. The pharmaceutical composition for use according to any one of Items 18 to 21, wherein the cancer cell is from a tumor carrying or expressing PD-L1, e.g. melanoma or a glioblastoma.
Item 23. Method for eliciting a CD4- or CD8-positive T-cell response in a subject independently from a presentation of an antigen by MHC, comprising administering to said subject the pharmaceutical composition for use according to any one of Items 1 to 8 or the pharmaceutical composition according to Item 18.
Item 24. A kit comprising; a) the pharmaceutical composition for use according to any one of Items 1 to 8 or the pharmaceutical composition according to Item 18, and b) a composition comprising at least one second active ingredient.
Item 25. The kit according to Item 24, wherein the second active ingredient is an anti-cancer agent, such as an anti-cancer agent selected from a chemotherapeutic agent, for example selected from: Actimide, Azacitidine, Azathioprine, Bleomycin, Carboplatin, Capecitabine, Cisplatin, Chlorambucil, Cyclophosphamide, Cytarabine, Daunorubicin, Docetaxel, Doxifluridine, Doxorubicin, Epirubicin, Etoposide, Fludarabine, Fluorouracil, Gemcitabine, Hydroxyurea, Idarubicin, Irinotecan, Lenalidomide, Leucovorin, Mechlorethamine, Melphalan, Mercaptopurine, Methotrexate, Mitoxantrone, Oxaliplatin, Paclitaxel, Pemetrexed, Revlimid, Temozolomide, Teniposide, Thioguanine, Valrubicin, Vinblastine, Vincristine, Vindesine, and Vinorelbine.
Item 26. The kit according to Item 25, where the provided compositions are to be administered simultaneously or sequentially.
Item 27. A method of preventing or treating a cancer cell exhibiting down-regulation or loss of HLA expression, wherein the cancer cell furthermore expresses PD-L1 (SEQ ID No. 7) or a protein having at least 75% sequence identity to PD-L1, preferably the PD-1-binding domain thereof, the method comprising administering to subject suffering or prospectively suffering from said cancer an effective amount of the pharmaceutical composition for use according to any one of Items 1 to 8, or of the pharmaceutical composition according to Item 18.
Item 28. The method according to Item 27, wherein said prevention or treatment comprises inducing a major histocompatibility complex (MHC)-independent T-cell response, preferably an MHC class I-independent T-cell response, or an MHC class I- and II-independent T-cell response.
Item 29. The method according to Item 27 or 28, wherein the cancer cell is from a tumor carrying or expressing PD-L1, e.g. melanoma or glioblastoma.
Item 30. The method according to any one of Items 27 to 29, wherein the method is combined with a further cancer treatment, wherein preferably the further treatment is selected from the group consisting of chemotherapy, radiotherapy, treatment with immunostimulating substances, gene therapy, treatment with antibodies and treatment using dendritic cells.

The invention will now be described further in the following examples with reference to the accompanying figures, nevertheless, without being limited thereto. For the purposes of the present invention, all references as cited are incorporated by reference in their entireties.

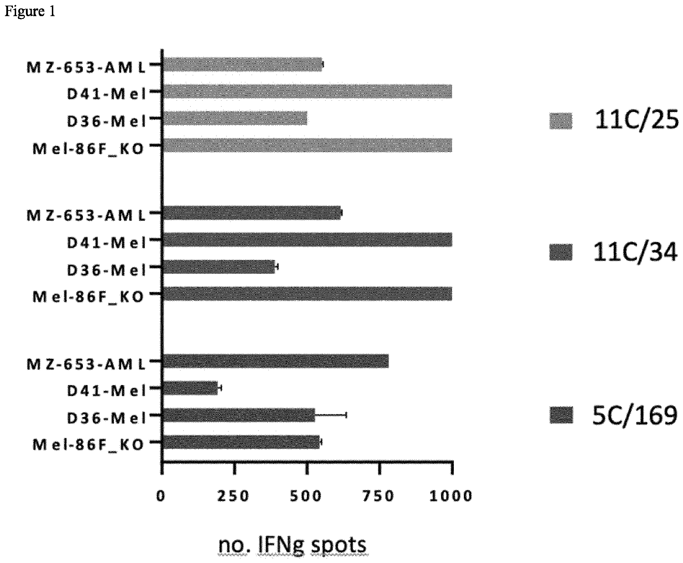

Figure 1 shows the cross reactivity patterns of T-cell clones (CTL) 11C/25, 11C/34 and 5C/169 according to the invention. CTL clones were tested in an IFNg-ELISPOT assay for recognition of allogeneic melanoma cell lines and primary AML blasts. All three clones recognized autologous melanoma cells (Mel-86F _KO lacking HLA I- and HLA II-surface expression due to spontaneous biallelic *B2M* deletions and *in vitro*-knockout of *CIITA*) and exhibited an identical or at least similar cross-reactivity pattern with allogeneic cell lines.

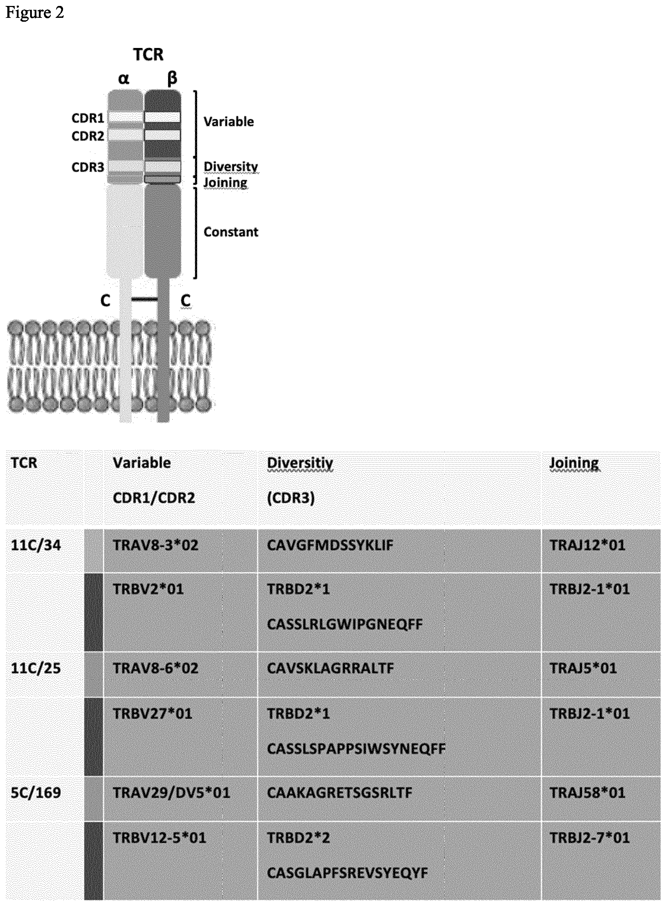

Figure 2 shows the cloning and sequencing of T-cell receptor (TCR) genes from CTL clones 11C/34, 11C/25 and 5C/169 according to the invention. To guarantee a constant source of T cells with the specificity of interest for library screening and further testing, the TCR alpha and beta chain genes from all three CTL clones were sequenced and cloned. For allele nomenclature and assignment see IMGT websites (https://www.imgt.org/IMGTrepertoire/ or https://www.imgt.org/IMGTindex/V-QUEST).

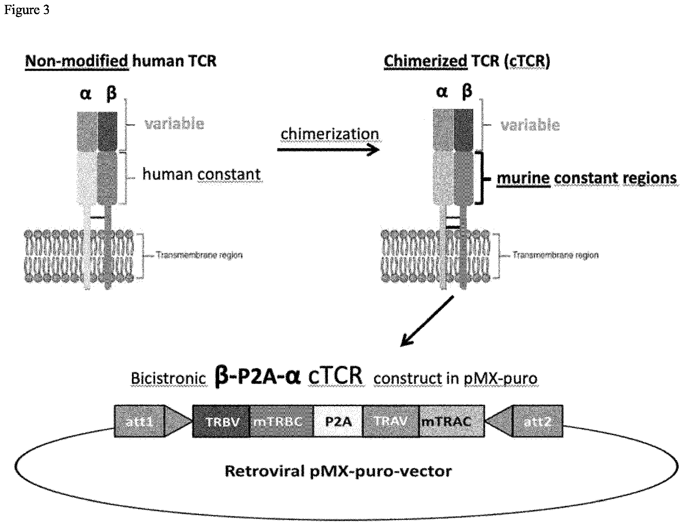

Figure 3 shows the bicistronic cloning and chimerization of αβTCRs. Variable regions (TRBV and TRAV) from TCR genes of all three CTL clones were cloned into a bicistronic vector backbone (doi: 10.1371/journal.pone.0238875). Accordingly, TCR optimizations comprised replacement of the human TCR constant regions by their codon-optimized murine counterparts [to generate chimeric TCR (cTCR)], addition of a second interchain disulfide bond and arrangement of the TCR chains in the order beta-P2A-alpha (doi: 10.1182/blood-2006-05-023069). Full amino acid and nucleotide sequences of chimerized TCR constructs according to the invention are given in Seq ID NOs 1 to 6.

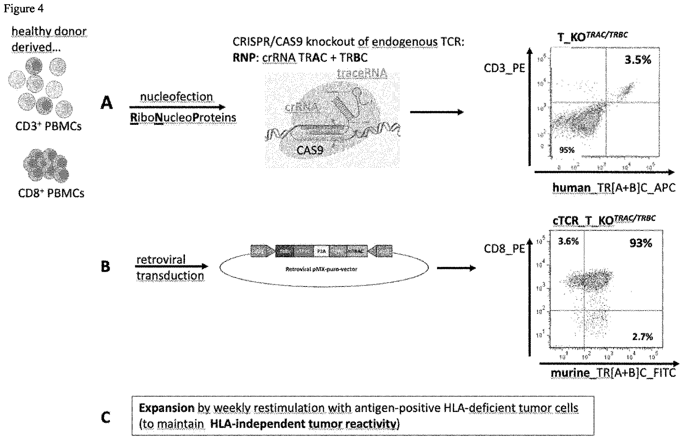

Figure 4 shows the generation and expansion of cTCR-transduced T cells from peripheral blood mononuclear cells (PBMC) of healthy donors. CD8-positive T cells were isolated from PBMC. Their endogenous TCR genes were knocked out via CRISPR/Cas9 (target gene segments were *TRAC* and *TRBC*) . Successful TCR knockout in T_KO^{*TRAC*/*TRBC*} cells was verified via flow cytometry using an antibody against the human TCR constant region (A). T_KO^{*TRAC*/*TRBC*} were retrovirally transduced with cTCR from CTL 11C/25, 11C/34 or 5C/169. Expression of the transduced cTCRs was verified via flow cytometry using an antibody against the murine TCR constant region (B). cTCR_T_KO^{*TRAC*/*TRBC*} were expanded via antigen-driven stimulation (C).

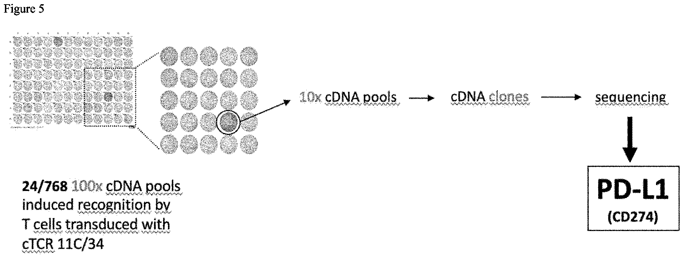

Figure 5 shows the library screening with the TCR from CTL 11C/34. A cDNA library constructed from AML blasts of patient MZ-653 was screened with T cells transduced with the TCR of CTL 11C/34 (see Figure 4). The cDNA library screening was performed as essentially described (doi: org/10.18632/oncotarget.16048), but was adapted to the needs for screening with allogeneic TCR-transduced T cells and for HLA-independent recognition. cDNA recipient cells were HEK293T cells that were HLA I/II-negative due to previous knockout of genes *B2M* and *CIITA* (HEK293T_KO^{*B2M*/*CIITA*}). Effector T cells were cTCR 11C/34_T_KO^{*TPAC*/*TRBC*} (see Figure 4). Antigen-coding cDNA (starting with 768 pools of 100 cDNA clones) was detected with IFNg-ELISPOT assays. Positive cDNA clones were found to encode PD-L1 (CD274). Shown are filters from wells containing target cells transfected with pools of 100 cDNA clones.

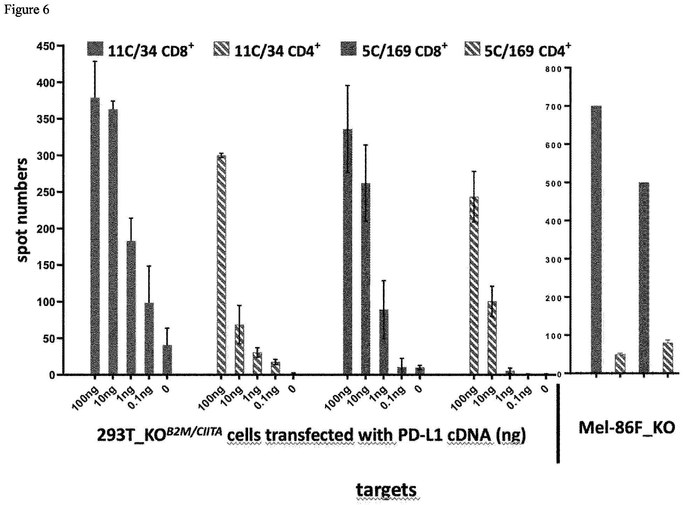

Figure 6 shows melanoma and PD-L1 reactivity of TCRs from CTL 11/34 and CTL 5C/169 in CD8- and in CD4-positive T cells. Shown are data of an IFNg-ELISPOT assay.

Recipient cells were HEK293T cells that were HLA I/II-negative due to previous knockout of genes *B2M* and *CIITA* (HEK293T_KO^{*B2M*/*CIITA*}) and were transiently transfected with titrated amounts of cDNA encoding PD-L1 (as indicated). Effector T cells were cTCR 11C/34_T_KO^{*TRAC*/*TRBC*} and cTCR 5C/169_T_KO^{*TRAC*/*TRBC*} cells (generated as described in Figure 4). Data as shown indicates that i) T cells transduced with cTCR from CTL clones 11C/34 and 5C/169 recognized HLA I/II-negative melanoma cells of patient Ma-MEL-86 (here: clone 86F with spontaneous biallelic *B2M* deletions and *in* vitro-knockout of *CIITA*), and cells transfected with PD-L1-encoding cDNA, and that ii) TCRs 11/34 and 5C/169 recognized PD-L1 better in CD8- than in CD4-positive T cells, which applied to all targets tested.

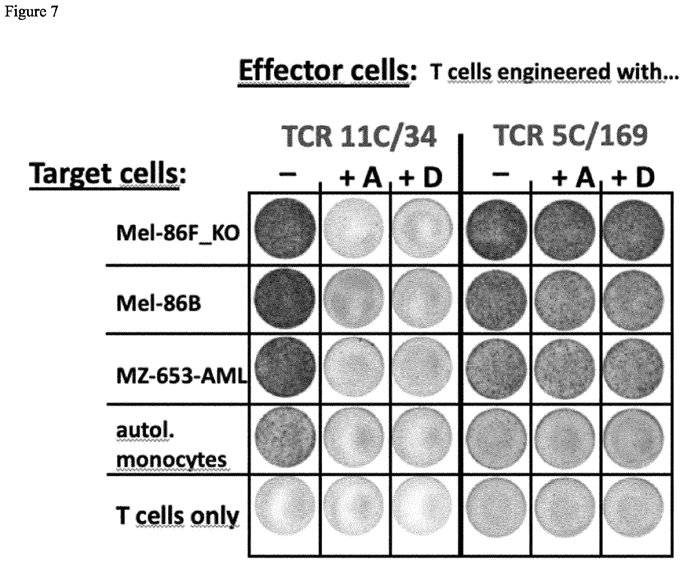

Figure 7 shows the blocking of T-cell recognition with PF-L1 inhibitors. Shown are data of an IFNg-ELISPOT assay. Effector T cells were cTCR11C/34_T_KO^{*TRAC*/*TRBC*} and cTCR5C/169_T_KO^{*TRAC*/*TRBC*} cells (generated as described in Figure 4). T cells transduced with TCRs 11/34 and 5C/169 recognized HLA-deficient melanoma cells of patient Ma-Mel-86 (here: clone 86F with spontaneous biallelic *B2M* deletions and *in vitro*-knockout of *CIITA,* clone 86B with spontaneous biallelic *B2M* deletion), and MZ-653-AML blasts. Rather weak recognition of autologous monocytes was observed for the cTCR 11C/34. Recognition by cTCR 11C/34 was inhibited by therapeutic anti-PD-L1 antibodies Atezolizumab (A) and Durvalumab (D), whereas recognition by T cells transduced with the TCR 5C/169 were not inhibited by any of the two anti-PD-L1 antibodies. These results indicate that TCR 11C/34 and TCR 5C/169 bind to different regions of PD-L1.

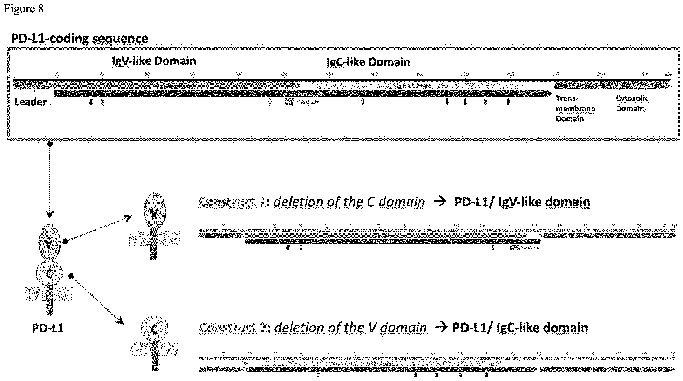

Figure 8 shows the generation of constructs encoding single PD-L1 domains. Schematic diagrams of full-length cDNA encoding PD-L1 and constructs 1 and 2 encoding single PD-L1 domains "IgV-like" (V) and "IgC-like" (C), respectively.

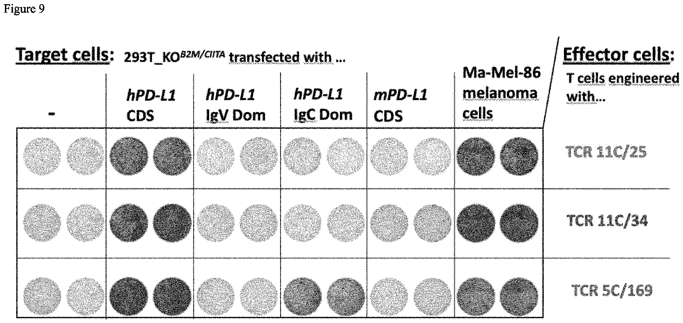

Figure 9 shows the fine specificity of anti-PD-L1 TCR. Data of an IFNg-ELISPOT assay. Targets were HLA I/II-negative 293T cells (293T_KO^{*B2M*/*CIITA*}) transfected with full-length human PD-L1 cDNA (hPD-L1 CDS), hPD-L1 domain-encoding cDNA fragments (Figure 8) or with full-length murine PD-L1 (mPD-L1), and Ma-Mel-86 melanoma cells. Data shown indicates that i) all three TCR recognize human PD-L1, but not its murine homologue, ii) recognition by TCR 11C/25 and TCR 11C/34 requires the presence of both the IgV and the IgC domain, iii) recognition by TCR 5C/169 only requires the presence of the IgC domain, and that iv) the TCR 5C/169 binds to a different region of PD-L1 compared to TCR 11C/25 and TCR 11C/34.

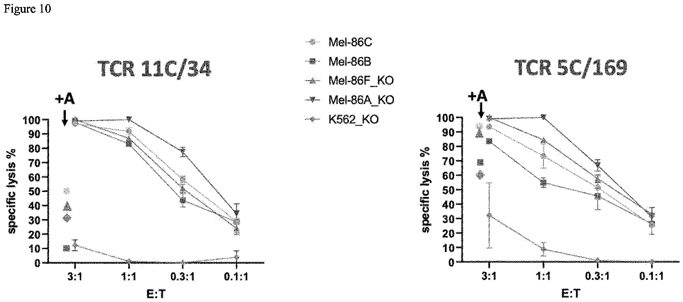

Figure 10 shows tumor-cell lysis by anti-PD-L1 TCR-engineered T cells. Data of a firefly luciferase cytolysis assay performed as described before (doi: 10.1371/journal.pone.0238875). Effectors were CD8+ buffy coat-derived T cells transduced with TCR 11C/34 and TCR 5C/169 (see Figure 4). Targets are explained in the figure. KO: double knockout of genes *B2M* and *CIITA*; A: Atezolizumab; E:T: effector-to-target cell ratio. Data shown indicates that i) HLA-independent anti-PD-L1 TCR 11C/34 and TCR 5C/169 mediate lytic activity, and that ii) lysis by TCR 11C/34 was inhibited by anti-PD-L1 antibody Atezolizumab, whereas lysis by TCR 5C/169 was not affected.

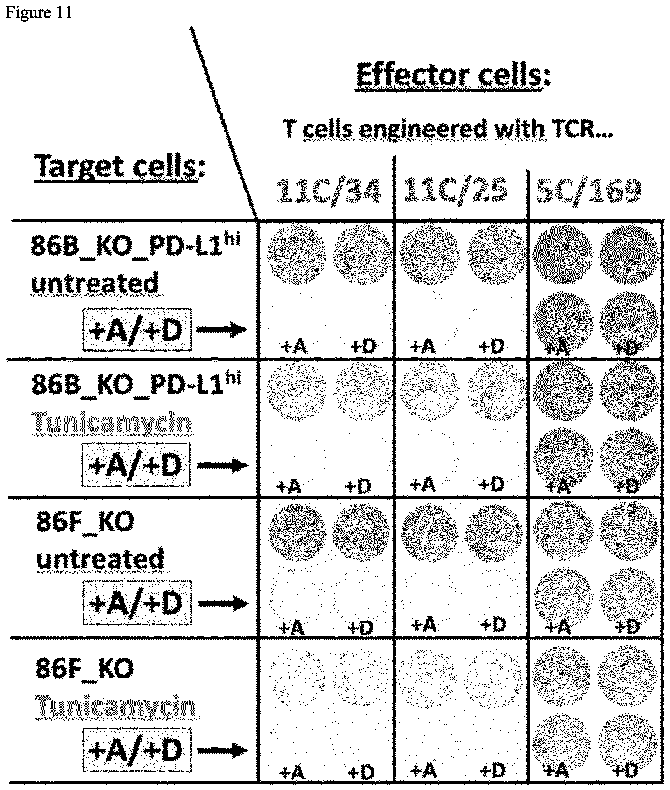

Figure 11 shows the influence of Tunicamycin pretreatment on recognition by anti-PD-L1 TCR-engineered T cells. Data of an IFNg-ELISPOT assay. Targets were HLA I/II-negative melanoma cell lines of patient Ma-Mel-86 (86B_KO_PD-L1^{hi} with spontaneous biallelic *B2M* deletions, *in vitro*-knockout of *CIITA* and stably transfected with PD-L1; 86F_KO with spontaneous biallelic *B2M* deletions and *in vitro*-knockout of *CIITA.* Targets were pretreated with N-glycosylation inhibitor tunicamycin where indicated. A: Atezolizumab; D: Durvalumab. Data shown indicates that i) deglycosylation with Tunicamycin reduced T-cell recognition by TCR 11C/25 and TCR 11C/34, and ii) Tunicamycin did not affect recognition by TCR 5C/169.

Seq ID NOs 1 and 2 show the full amino acid and nucleotide sequence, respectively, of chimerized TCR construct 11C/25 according to the invention.

Seq ID NOs 3 and 4 show the full amino acid and nucleotide sequence, respectively, of chimerized TCR construct 11C/34 according to the invention.

Seq ID NOs 5 and 6 show the full amino acid and nucleotide sequence, respectively, of chimerized TCR construct 5C/169 according to the invention.

SEQ ID No. 7 shows the amino acid sequence of human PD-L1 (uniport database Q9NZQ7).

### Examples

In earlier work of the inventors, patient-derived, HLA-independent, anti-tumor T-cell receptors (TCR) directed against tyrosinase-related protein 2 (TRP2) and the GM-CSF receptor alpha chain (CSF2RA) were identified. In vivo anti-tumor effects after adoptive transfer were determined so far for an MHC-independent TCR against human and murine TRP2, while hitherto no unwanted off-tumor and off-target effects were seen.

In the context of the present invention, a further HLA-independent, tumor-associated T-cell target was identified in the form of PD-L1. This was achieved via library expression screening with T cells transduced with TCR cloned from HLA-independent antitumoral T-cell clones of unknown specificity.

In melanoma patient Ma-Mel-86 the inventors have observed that over years the anti-tumor T-cell repertoire was increasingly dominated by T cells that recognized autologous melanoma cells in an HLA-independent manner. Altogether the inventors identified six TCR clonotypes belonging to the HLA-independent sub-repertoire. The target antigens for three of them have been identified before. Two are directed against tyrosinase-related protein 2 (TRP2) and one against the GM-CSF receptor alpha chain (CSF2RA) (see WO2014091034A1). Two of the clonotypes (one against CSF2RA and one against a so far unknown target antigen) strongly prevailed within the HLA-independent sub-repertoire in peripheral blood.

The inventors now identified the target antigen of the other three clonotypes - assigned to CTL clones 11C/25, 11C/34 and 5C/169. These CTL clones did not recognize any of the above-mentioned antigens and exhibited a rather similar cross-reactivity pattern (on allogeneic melanoma cell lines and primary acute myeloid leukemia - AML - cells). Their TCR have been cloned, chimerized with murine constant TCR regions (to obtain cTCR) and finally provided in bicistronic retroviral constructs. The cTCR constructs were transduced into blood-derived T lymphocytes from healthy donors after CRISPR/Cas9-mediated knockout of endogenous TCR to avoid mispairing (cTCRX/Y-T_KOTRAC,TRBC).

### Results

T cells transduced with the cTCR from CTL11/34 (cTCR11/34-T_KO^{*TRAC*,*TRBC*}) were used for a cDNA expression screening of a cDNA library prepared from primary AML blasts (MZ653-AML) (Figure 5).

Recipient cells were HEK293T cells lacking surface HLA molecules due to CRISPR/Cas9 knockout of the genes B2M and CIITA (HEK293T_KO^{*B2M*/*CIITA*}). In the cDNA library from MZ653-AML blasts antigen-encoding cDNA was identified, cloned and sequenced. It was found to encode the immune checkpoint molecule PD-L1 (CD274). All three cTCR within hitherto unknown specificity (derived from CTL clones 11C/25, 11C/34 and 5C/169) were found to recognize PD-L1 (Figures 6 and 9).

Further characterization of PD-L1 recognition by patient-derived HLA-independent clonotypes revealed that
i) all identified anti-PD-L1 cTCR mediate lysis (as seen in bioluminescence assays, Figure 10);
ii) CD8+ and CD4+ T cells engineered with anti-PD-L1 cTCR are both functional, CD8+ cells were found to be superior to CD4+ cells (Figure 6);
iii) none of the three cTCR cross-reacted with murine PD-L1 (Figure 9);
iv) anti-PD-L1 cTCRs of patient Ma-Mel-86 reacted with at least two distinct PD-L1 domains (Figure 9).

Recognition of the cTCR 11C/34 and 11C/25 is inhibited by Atezolizumab and Durvalumab (two PD-L1 inhibitors in routine clinical use) (Figures 7 and 11), is clearly N-glycosylation dependent (i.e. recognition is tunicamycin-sensitive) (Figure 11), and requires the presence of the IgV-like domain of PD-L1 (Figure 9).

Recognition of the clone cTCR 5C/169 is not inhibited by Atezolizumab and Durvalumab, is not tunicamycin-sensitive, requires the presence of the IgC-like domain of PD-L1 alone (Figures 7, 9 and 11).

### Literature as cited

Horn LA, Ciavattone NG, Atkinson R, Woldergerima N, Wolf J, Clements VK, Sinha P, Poudel M, Ostrand-Rosenberg S. CD3xPDL1 bi-specific T cell engager (BiTE) simultaneously activates T cells and NKT cells, kills PDL1(+) tumor cells, and extends the survival of tumor-bearing humanized mice. Oncotarget. 2017 Aug 3;8(35):57964-57980. doi: 10.18632/oncotarget.19865.
Jiang W, Li T, Guo J, Wang J, Jia L, Shi X, Yang T, Jiao R, Wei X, Feng Z, Tang Q, Ji G. Bispecific c-Met/PD-L1 CAR-T cells have enhanced therapeutic effects on hepatocellular carcinoma. Front Oncol. 2021 Mar 10;11:546586. doi: 10.3389/fonc.2021.546586.
Khalique H, Baugh R, Dyer A, Scott EM, Frost S, Larkin S, Lei-Rossmann J, Seymour LW. Oncolytic herpesvirus expressing PD-L1 BiTE for cancer therapy: exploiting tumor immune suppression as an opportunity for targeted immunotherapy. J Immunother Cancer. 2021 Mar;9(4):e001292. doi: 10.1136/jitc-2020-001292.
Kjeldsen JW, Lorentzen CL, Martinenaite E, Ellebaek E, Donia M, Holmstroem RB, Klausen TW, Madsen CO, Ahmed SM, Weis-Banke SE, Holmström MO, Hendel HW, Ehrnrooth E, Zocca MB, Pedersen AW, Andersen MH, Svane IM. A phase 1/2 trial of an immune-modulatory vaccine against IDO/PD-L1 in combination with nivolumab in metastatic melanoma. Nat Med. 2021 Dec;27(12):2212-2223. doi: 10.1038/s41591-021-01544-x.
Li D, English H, Hong J, Liang T, Merlino G, Day CP, Ho M. A novel PD-L1-targeted shark V(NAR) single-domain-based CAR-T cell strategy for treating breast cancer and liver cancer. Mol Ther Oncolytics. 2022 Feb 20;24:849-863. doi: 10.1016/j.omto.2022.02.015.
Liu M, Wang X, Li W, Yu X, Flores-Villanueva P, Xu-Monette ZY, Li L, Zhang M, Young KH, Ma X, Li Y. Targeting PD-L1 in non-small cell lung cancer using CAR T cells. Oncogenesis. 2020 Aug 13;9(8):72. doi: 10.1038/s41389-020-00257-z. Mansilla-Soto J, Eyquem J, Haubner S, Hamieh M, Feucht J, Paillon N, Zucchetti AE, Li Z, Sjöstrand M, Lindenbergh PL, Saetersmoen M, Dobrin A, Maurin M, Iyer A, Garcia Angus A, Miele MM, Zhao Z, Giavridis T, van der Stegen SJC, Tamzalit F, Rivière I, Huse M, Hendrickson RC, Hivroz C, Sadelain M. HLA-independent T cell receptors for targeting tumors with low antigen density. Nat Med. 2022 Feb;28(2):345-352. doi: 10.1038/s41591-021-01621-1.
Schrörs B, Lübcke S, Lennerz V, Fatho M, Bicker A, Wölfel C, Derigs P, Hankeln T, Schadendorf D, Paschen A, Wölfel T. HLA class I loss in metachronous metastases prevents continuous T cell recognition of mutated neoantigens in a human melanoma model. Oncotarget. 2017 Apr 25;8(17):28312-28327. doi: 10.18632/oncotarget.16048. PMID: 28423700; PMCID: PMC5438652.
Tran E, Robbins PF, Lu YC, Prickett TD, Gartner JJ, Jia L, Pasetto A, Zheng Z, Ray S, Groh EM, Kriley IR, Rosenberg SA. T-Cell Transfer Therapy Targeting Mutant KRAS in Cancer. N Engl J Med. 2016 Dec 8;375(23):2255-2262. doi: 10.1056/NEJMoa1609279. PMID: 27959684; PMCID: PMC5178827.

## Claims

1. A binding agent comprising the amino acid sequence of a binding region of a T-cell receptor binding to a cancer cell expressing PD-L1 (SEQ ID No. 7) or a protein having at least 75% sequence identity to PD-L1, preferably of the PD-1-binding domain thereof, wherein said binding is independent of HLA.

2. The binding agent according to claim 1, wherein said T-cell receptor is selected from a T-cell receptor expressed by one of the CTL clones 11C/25, 11C/34 and 5C/169, or an amino acid sequence of a TCR alpha or beta chain selected from a sequence according to SEQ ID NO: 1, 3, and 5.

3. A nucleic acid molecule encoding a binding agent according to claims 1 or 2, a vector comprising the nucleic acid molecule, such as, for example, an expression vector comprising and expressing the nucleic acid molecule, or a recombinant or genetically modified cell comprising the nucleic acid molecule or vector.

4. A recombinant or genetically modified T cell expressing a TCR that binds to PD-L1 or a protein having at least 75% sequence identity to PD-L1, preferably of the PD-1-binding domain thereof, wherein said binding is independently from HLA, such as, for example, a TCR expressed by one of the CTL clones 11C/25, 11C/34 or 5C/169 according to SEQ ID NO: 1, 3 or 5, respectively, or a PD-L1 binding fragment thereof.

5. An *in-vitro* method for generating HLA independent T cells, comprising
a) providing a first cell that expresses PD-L1 (SEQ ID No. 7) or a protein having at least 75% sequence identity to PD-L1 or an immunogenic protein fragment thereof, preferably wherein the polypeptide is full length PD-L1 or preferably the PD-1-binding domain,
b) bringing a population of peripheral blood mononuclear cells (PBMCs) into contact with said first cell, and thereby stimulating said PBMCs, and
c) selecting from the population of stimulated PBMCs T cells that have the ability to recognize a cell expressing the PD-L1 or an immunogenic protein fragment thereof used in a) independently of the expression of HLA in said cell, wherein preferably in step c) the ability of a T cell to recognize a cell expressing the PD-L1 or an immunogenic protein fragment thereof used in a) independently of the expression of HLA in said cell is determined by testing the reactivity of said T-cell against a cell expressing the PD-L1 or an immunogenic protein fragment thereof, wherein
i) the cell expressing the PD-L1 or an immunogenic protein fragment thereof lacks MHC class I or MHC class I and II expression, and/or
ii) the T-cell is tested for its reactivity against said cell expressing the PD-L1 or an immunogenic protein fragment thereof in the presence of antibodies against MHC class I or II; and/or
iii) the T-cell is tested for its reactivity against xenogenic cells transfected with DNA or RNA encoding the PD-L1 or an immunogenic protein fragment thereof,
wherein in i), ii) and/or iii) a T-cell that shows reactivity is a T-cell having the ability to recognize a cell expressing the PD-L1 or an immunogenic protein fragment thereof used in a) independent of the expression of HLA in said cell.

6. An HLA-independent T cell, generated according to a method according to claim 5.

7. A pharmaceutical composition, comprising a binding agent according to any one of claims 1 or 2, a nucleic acid or vector according to claim 3, or a T-cell according to claim 4, and at least one pharmaceutically acceptable carrier, preferably the pharmaceutical composition for use as a medicament, preferably for use in the prevention or treatment of a cancer cell exhibiting down-regulation or loss of HLA expression, wherein preferably said prevention or treatment comprises inducing a major histocompatibility complex (MHC)-independent T-cell response, preferably an MHC class I-independent T-cell response, or an MHC class I- and II-independent T-cell response.

8. The pharmaceutical composition for use according to claim 7, wherein the cancer cell expresses PD-L1 (SEQ ID No. 7) or a protein having at least 75% sequence identity to PD-L1, preferably the PD-1-binding domain thereof.

9. A pharmaceutical composition comprising PD-L1 of SEQ ID NO: 7 or a functional homologue thereof at least 75% identical thereto or an immunogenically active peptide fragment comprising a consecutive sequence of at least 8 amino acids of PD-L1 or said functional homologue thereof ,preferably of the PD-1-binding domain thereof, or a nucleic acid encoding said PD-L1, said functional homologue thereof or said peptide fragment; and b) at least one pharmaceutically acceptable carrier for use in the prevention or treatment of a cancer cell exhibiting down-regulation or loss of HLA expression, which preferably is an anti-cancer vaccine.

10. The pharmaceutical composition for use according to claim 9, wherein said prevention or treatment comprises inducing a major histocompatibility complex (MHC)-independent T-cell response, preferably an MHC class I-independent T-cell response, or an MHC class I- and II-independent T-cell response, wherein preferably the cancer cell expresses PD-L1 (SEQ ID No. 7) or a protein having at least 75% sequence identity to PD-L1, preferably the PD-1-binding domain thereof.

11. The pharmaceutical composition for use according to claim 9 or 10, wherein the vaccine composition comprises antigen-presenting cells comprising the immunogenically active peptide fragment or a nucleic acid encoding said immunogenically active peptide fragment, wherein preferably the antigen-presenting cell is a dendritic cell.

12. Method for eliciting a CD4- or CD8-positive T-cell response in a subject independently from a presentation of an antigen by MHC, comprising administering to said subject the pharmaceutical composition for use according to any one of claims 7 to 11.

13. A kit comprising; a) the pharmaceutical composition for use according to any one of claims 7 to 11, and b) a composition comprising at least one second pharmaceutically active ingredient.

14. The kit according to claim 13, wherein the second active ingredient is an anti-cancer agent, such as an anti-cancer agent selected from a chemotherapeutic agent, for example selected from: Actimide, Azacitidine, Azathioprine, Bleomycin, Carboplatin, Capecitabine, Cisplatin, Chlorambucil, Cyclophosphamide, Cytarabine, Daunorubicin, Docetaxel, Doxifluridine, Doxorubicin, Epirubicin, Etoposide, Fludarabine, Fluorouracil, Gemcitabine, Hydroxyurea, Idarubicin, Irinotecan, Lenalidomide, Leucovorin, Mechlorethamine, Melphalan, Mercaptopurine, Methotrexate, Mitoxantrone, Oxaliplatin, Paclitaxel, Pemetrexed, Revlimid, Temozolomide, Teniposide, Thioguanine, Valrubicin, Vinblastine, Vincristine, Vindesine, and Vinorelbine, wherein preferably the provided compositions are to be administered simultaneously or sequentially.

15. A method of preventing or treating a cancer cell exhibiting down-regulation or loss of HLA expression, wherein the cancer cell furthermore expresses PD-L1 (SEQ ID No. 7) or a protein having at least 75% sequence identity to PD-L1, preferably the PD-1-binding domain thereof, the method comprising administering to subject suffering or prospectively suffering from said cancer an effective amount of the pharmaceutical composition for use according to any one of claims 7 to 11, wherein preferably the method is combined with a further cancer treatment, wherein preferably the further treatment is selected from the group consisting of chemotherapy, radiotherapy, treatment with immunostimulating substances, gene therapy, treatment with antibodies and treatment using dendritic cells.
